# EUROPEAN PATENT SPECIFICATION

(11) **EP 4 519 264 B1**
(45) Date of publication and mention of the grant of the patent: **29.04.2026**
(21) Application number: 23723886.0
(22) Date of filing: 28.04.2023
(51) Int. Cl.: C07D 471/08, A61P 25/00, A61K 31/55

(54) **NICOTINIC ACETYLCHOLINE RECEPTOR LIGANDS**
NIKOTINACETYLCHOLINREZEPTOR-LIGANDEN
LIGANDS DU RÉCEPTEUR DE L'ACÉTYLCHOLINE NICOTINIQUE

(30) Priority: 05.05.2022 EP 22171886
(43) Date of publication of application: 12.03.2025
(73) Proprietor: Philip Morris Products S.A., 2000 Neuchâtel (CH)
(72) Inventor: HOENG, Julia, 2000 Neuchâtel (CH); HO, Jenny, Redhill Singapore 159471 (SG); LOW, Si Ling Tiffany, Redhill Singapore 159471 (SG); MAZUROV, Anatoly, Greensboro, North Carolina 27410 (US)
(74) Representative: Reddie & Grose LLP
(86) International application number: PCT/EP2023/061390
(87) International publication number: WO 2023/213739

(56) References cited:
- WO-A1-2004/013137
- WO-A1-2006/065217
- WO-A1-2016/154434
- WO-A1-98/54152
- W.VON DOERING, LUDMILA BIRLADEANU, D.A.ANDREWS AND M. PAGNOTTA: "Conjugative Interaction in the Orthogonal Enamine,l-Azabicyclo[3.2.2]non-2-ene", J.AM.CHEM.SOC., vol. 107, 1985, pages 428 - 432, XP055968863

## Description

### Background

Nicotinic acetylcholine receptors (nAChRs) have been extensively characterized for their function in peripheral and central neurons where they play a role in modulating neurotransmission. nAChRs typically associated with the central nervous system (CNS) have been shown to occur in several subtypes. The most common nAChR subtypes are α4β2 and α7 subtypes. α7 nAChRs belong to the family of acetylcholine-gated cation channels and exhibit distinct biophysical and pharmacological profiles relative to other nAChR subtypes.

Although these receptors are predominantly expressed in neuronal tissues, several types of immune cells also express α7 nAChR mRNA. Examples for such immune cells expressing α7 nAChR mRNA are macrophages, T-cells, B-cells, microglia, monocytes and dendritic cells. Immunological responses to protect against excessive inflammation can be regulated by the CNS through the cholinergic anti-inflammatory pathway, in which acetylcholine is released from vagus nerves. Consequently, acetylcholine activates the post-synaptic α7 nAChR of innervated tissues including splenic nerves following its stimulation and in turn inhibits inflammatory cytokines.

Various nicotinic compounds have been known to interact with α7 nAChR and thus have been proposed for therapy. However, a disadvantage of those known nicotinic compounds is that they are associated with various undesirable side effects, for example, by stimulating muscle and ganglionic receptors.

In turn, there is a need for compounds, compositions, and methods for preventing or treating various conditions or disorders, such as CNS disorders, including alleviating the symptoms of these disorders but without the associated side effects. More specifically, there is a need for compounds, compositions, and methods that affect CNS function without significantly affecting those nicotinic receptor subtypes which have the potential to induce undesirable side effects, such as appreciable activity at cardiovascular and skeletal muscle sites.

### Summary of Invention

According to an aspect of the invention, there is provided a compound of general formula (I): 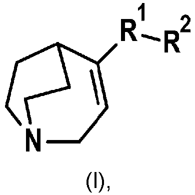 or a pharmaceutically acceptable salt thereof,
wherein:
R¹ is an optionally substituted aryl or an optionally substituted heteroaryl; and
R² is a halogen, a triflate, an optionally substituted aryl, an optionally substituted heteroaryl or is absent.

Also provided are a pharmaceutical composition comprising the compound of general formula (I), uses of the compound of general formula (I) uses of the composition comprising the compound of general formula (I), and methods of manufacturing the compound of general formula (I) as defined in the appended independent claims, to which reference should now be made. Preferred or advantageous features of the invention are set out in dependent claims and described further below.

The compounds of general formula (I) of the invention advantageously affect the central nervous system without inducing potentially undesirable side effects of nicotinic receptor subtypes. These potentially undesirable side effects may include appreciable activity at cardiovascular and skeletal muscle sites.

More specifically, the compounds of general formula (I) of the present invention are α7 nAChR agonists, which provide a novel, non-narcotic approach to therapy in patients with acute and chronic cough. As described below, potential application of selective α7 nAChR agonists as antitussive agents according to the invention was demonstrated in *in vivo* experiments by inhibiting cough responses in guinea pigs evoked by citric acid.

In addition, α7 nAChR agonists as described herein might be beneficial for the treatment of cough origins. In preclinical studies of pulmonary disease, α7 nAChR agonists demonstrated anti-inflammatory effects in models of acute lung injury, allergen challenge and gram-negative bacterial infection.

According to an aspect of the invention, there is provided a compound of general formula (I): 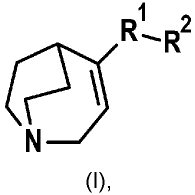 or a pharmaceutically acceptable salt thereof. R¹ is an optionally substituted aryl or an optionally substituted heteroaryl. R² is a halogen, a triflate, an optionally substituted aryl, an optionally substituted heteroaryl or is absent.

The term "halo" or "halogen" as used herein refers to any radical of fluorine, chlorine, bromine or iodine.

The term "aryl" as used herein refers to monocyclic, bicyclic or tricyclic aromatic groups containing from 6 to 14 carbon atoms in the ring. Common aryl groups include C₆-C₁₄ aryl, for example, C₆-C₁₀ aryl. Non-limiting examples of C₆-C₁₄ aryl groups include phenyl, naphthyl, phenanthrenyl, anthracenyl, indenyl, azulenyl, biphenyl, biphenylenyl and fluorenyl groups. An "optionally substituted aryl" group may include the substituents as described herein.

The term "heteroaryl" as used herein refers to aromatic groups having 5 to 14 ring atoms (for example, 5 to 10 ring atoms) and containing carbon atoms and 1, 2 or 3 oxygen, nitrogen or sulfur heteroatoms. Examples of heteroaryl groups include thienyl (thiophenyl), benzo[b]thienyl, naphtho[2,3-b]thienyl, thianthrenyl, furyl (furanyl), benzofuranyl, isobenzofuranyl, chromenyl, xanthenyl, phenoxanthiinyl, pyrrolyl, including without limitation 2H-pyrrolyl, imidazolyl, pyrazolyl, triazolyl, tetrazolyl, pyridyl (pyridinyl), including without limitation 2-pyridyl, 3-pyridyl, and 4-pyridyl, pyrazinyl, pyrimidinyl, pyridazinyl, triazinyl, tetrazinyl, indolizinyl, isoindolyl, 3H-indolyl, indolyl, indazolyl, purinyl, 4H-quinolizinyl, isoquinolyl, quinolyl, phthalazinyl, naphthyridinyl, quinozalinyl, cinnolinyl, pteridinyl, carbazolyl, β-carbolinyl, phenanthridinyl, acrindinyl, perimidinyl, phenanthrolinyl, phenazinyl, isothiazolyl, phenothiazinyl, isoxazolyl, furazanyl, and phenoxazinyl. Where the heteroaryl group contains a nitrogen atom in a ring, such nitrogen atom may be in the form of an N-oxide, e.g., a pyridyl N-oxide, pyrazinyl N-oxide and pyrimidinyl N-oxide. An "optionally substituted heteroaryl" group may include the substituents as described herein.

Optionally, the or each aromatic ring of the carbocyclic or heterocyclic aromatic ring of one or both aryl groups of general formula (I); the aryl of the optionally substituted aryl of general formula (I); and the heteroaryl of the optionally substituted heteroaryl of general formula (I) is a 3- to 10-membered ring, for example a 5- to 10-membered ring. For example, the or each aromatic ring of the carbocyclic or heterocyclic aromatic ring of one or both aryl groups of general formula (I); the aryl of the optionally substituted aryl of general formula (I); and the heteroaryl of the optionally substituted heteroaryl of general formula (I) may be a 3-membered ring, 4-membered ring, 5-membered ring, 6-membered ring, 7-membered ring, 8-membered ring, 9-membered ring, or 10-membered ring.

Optionally, one or both aryl groups of general formula (I); and each aryl or heteroaryl of general formula (I) are monocyclic. For example, the one or both aryl groups of general formula (I); and each aryl or heteroaryl of general formula (I) may be independently selected from the group consisting of: phenyl, furanyl, pyrrolyl, thienyl, pyridinyl, pyrimidinyl, pyridazinyl, pyrazinyl, oxazolyl, oxadiazolyl, isoxazolyl, pyrazolyl, imidazolyl, thiazolyl, isothiazolyl and thiadiazolyl. In one embodiment, R¹ does not include phenyl.

Optionally, one or both aryl groups of general formula (I); and each aryl or heteroaryl of general formula (I) are polycyclic. For example, the one or both aryl groups of general formula (I); and each aryl or heteroaryl of general formula (I) may be independently selected from the group consisting of: naphthalenyl, anthracenyl, indolizinyl, indolyl, isoindolyl, benzofuranyl, benzothiophenyl, benzodioxolanyl, indazolyl, pyrrolopyridinyl, benzimidazolyl, benzothiazolyl, benzoisothiazolyl, purinyl, thienopyrazinyl, quinolinyl, isoquinolinyl, cinnolinyl, phthalazinyl, quinazolinyl, quinoxalinyl, 1,8-naphthyridinyl, pteridinyl, carbazolyl, acridinyl, naphtiridinyl, phenazinyl, phenothiazinyl, phenoxazinyl and azulenyl.

Optionally, one or both aryl groups of general formula (I); and each aryl or heteroaryl of general formula (I) are independently selected from the group consisting of: acenaphthylene, acephenanthrylene, acridine, anthanthrene, anthracene, 9,10-anthracenedione, 9(10H)-anthracenone, anthraquinone, anthrone, benz[e]acephenanthrylene, benz[c]acridine, benz[a]anthracene, 7H-benz[de]anthracen-7-one, benzanthrone, benzo[b]chrysene, benzo[c]chrysene, benzo[g]chrysene, benzo[c]cinnoline, benzo[a]dibenzothiophene, benzo[b]fluoranthene, benzo[ghi]fluoranthene, benzo[j]fluoranthene, benzo[k]fluoranthene, 11H-benzo[a]fluorene, 11H-benzo[b]fluorene, 7H-benzo[c]fluorene, benzo[h]naphtho [1,2-f]quinolene, benzo[b]naphtho[2, 1-d]thiophene, benzo[rst]pentaphene, benzo[ghi]perylene, benzo[c]phenanthrene, benzo[a]pyrene, benzo[e]pyrene, benzo[f]quinoline, benzo[h]quinoline, benzo[b]triphenylene, biphenylene, 9H-carbazole, chrysene, coronene, 4H-cyclopenta[def]phenanthrene, cyclopenta[cd]pyrene, dibenz[a,h]acridine, dibenz[a,j]acridine, dibenz[c,h]acridine, dibenz[a,c]anthracene, dibenz[a,h]anthracene, dibenz[a,j]anthracene, 7H-dibenzo[a,g]carbazole, 13H-dibenzo[a,i]carbazole, 7H-dibenzo[c,g]carbazole, dibenzo[b,def]chrysene, dibenzo[def,mno]chrysene, dibenzo[def,p]chrysene, dibenzo[b,h]phenanthrene, dibenzo[a,e]pyrene, dibenzo[a,h]pyrene, dibenzo[a,i]pyrene, dibenzo[a,1]pyrene, dibenzothiophene, fluoranthene, 9H-fluorene, 9H-fluoren-9-one, indeno[1,2,3-cd]pyrene, 1H-indole, isoquinoline, naphthacene, naphthalene, naphtho[1,2,3,4-def] chrysene, naphtho[2,3-f]quinoline, pentaphene, perylene, 1H-phenalene, phenanthraquinone, phenanthrene, 9,10-phenanthrenedione, phenanthridine, 1,10-phenanthroline, phenanthro[4,5-bcd]thiophene, phenazine, phenazone, picene, pyrene, quinoline, triphenylene and 9H-xanthene.

Optionally, one or both aryl groups of general formula (I); and each aryl or heteroaryl of general formula (I) are substituted, for example with 1, 2 or 3 substituents.

Optionally, the substituents are independently selected from the group consisting of: alkyl, alkenyl, heterocyclyl, cycloalkyl, aryl, substituted aryl, arylalkyl, substituted arylalkyl, halo (for example, F, Cl, Br or I), --OR', --NR'R", --CF3, --CN, --NO₂, --SR', --N₃, --C(=O)NR'R", --NR'C(=O)R", --C(=O)R',-C(=O)OR', --OC(=O)R', --O(CR'R"), --C(=O)R', --SO₂R', and --SO₂NR'R", wherein R' and R" are individually hydrogen, lower alkyl. The lower alkyl may be methyl, ethyl, propyl, isopropyl, butyl, or t-butyl. For example, the substituents may be straight chain or branched alkyl including C₁-C₈, preferably C₁-C₈, such as methyl, ethyl or isopropyl, cycloalkyl, heterocyclyl, aryl, or arylalkyl (such as benzyl).

Optionally, R' and R" combine to form a cyclic functionality.

Optionally, the substituents are independently selected from the group consisting of: cyano, halo, alkyl, haloalkyl, cycloalkyl, alkoxy, haloalkoxy and alkylthio.

Optionally, R¹ of general formula (I) is selected from: 1,3,4-thiadiazolyl, pyridinyl, pyridazinyl, pyrimidinyl and pyrazinyl.

Optionally, R² of general formula (I) is selected from indolyl, benzofuranyl, benzothiazolyl and phenyl.

Optionally, one or both aryl groups of general formula (I) may comprise a carbocyclic or heterocyclic aromatic ring. Optionally one or both aryl groups of general formula (I) may be an optionally substituted carbocyclic or heterocyclic aromatic ring.

Optionally, the compound is selected from the group consisting of:
4-(3-pyridyl)-1-azabicyclo[3.2.2]non-3-ene;
4-(6-phenyl-3-pyridyl)-1-azabicyclo[3.2.2]non-3-ene; and
4-(6-phenylpyridazin-3-yl)-1-azabicyclo[3.2.2]non-3-ene.

Advantageously, 4-(6-phenyl-3-pyridyl)-1-azabicyclo[3.2.2]non-3-ene significantly reduces the citric acid-induced cough counts when compared with saline control.

Further, 4-(6-phenyl-3-pyridyl)-1-azabicyclo[3.2.2]non-3-ene also showed significant improvement in cough incubation period at dosage approximately 20 to 30-fold lower than codeine which is considered as the "gold standard" narcotic cough suppressant.

Thus, in a preferred embodiment of the invention, there is provided: 4-(6-phenyl-3-pyridyl)-1-azabicyclo[3.2.2]non-3-ene or a pharmaceutically acceptable salt thereof.

In a preferred embodiment of the invention, there is provided a compound of the following formula or a pharmaceutically acceptable salt thereof:

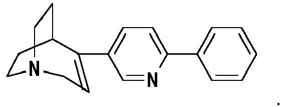

Optionally, the pharmaceutically acceptable salt is selected from one or more of the following: chloride, bromide, sulfate, phosphate, and nitrate; organic acid addition salts such as acetate, galactarate, propionate, succinate, lactate, glycolate, malate, tartrate, citrate, maleate, fumarate, methanesulfonate, p-toluenesulfonate, ascorbate; salts with acidic amino acids, for example aspartate or glutamate, or hydrates or ethanol solvates.

Optionally, the compounds of general formula (I) may exist in unsolvated forms as well as solvated forms, including hydrated forms. "Hydrate" refers to a complex formed by combination of water molecules with molecules or ions of the solute. "Solvate" refers to a complex formed by combination of solvent molecules with molecules or ions of the solute. The solvent may be an organic compound, an inorganic compound, or a mixture of both. Solvate is meant to include hydrate. Some examples of solvents include, but are not limited to, methanol, acetonitrile, N,N-dimethylformamide, tetrahydrofuran, dimethylsulfoxide, and water. In general, the solvated forms are equivalent to unsolvated forms and are encompassed within the scope of the present invention. Optionally, the compounds of general formula (I) may exist as solid material in e.g. multiple crystalline or amorphous forms. In general, all physical forms are equivalent for the uses contemplated by the present invention and are intended to be within the scope of the present invention.

"Tautomer" means compounds produced by the phenomenon wherein a proton of one atom of a molecule shifts to another atom (See, Jerry March, Advanced Organic Chemistry: Reactions, Mechanisms and Structures, Fourth Edition, John Wiley & Sons, pages 69-74 (1992)). The tautomers also refer to one of two or more structural isomers that exist in equilibrium and are readily converted from one isomeric form to another. The compounds described herein may have one or more tautomers and therefore include various isomers. All such isomeric forms of these compounds are expressly included in the present invention.

"Isomers" mean compounds having identical molecular formulae but differ in the nature or sequence of bonding of their atoms or in the arrangement of their atoms in space. Isomers that differ in the arrangement of their atoms in space are termed "stereoisomers". "Stereoisomer" and "stereoisomers" refer to compounds that exist in different stereoisomeric forms if they possess one or more asymmetric centres or a double bond with asymmetric substitution and, therefore, may be produced as individual stereoisomers or as mixtures. Stereoisomers include enantiomers and diastereomers. Stereoisomers that are not mirror images of one another are termed "diastereomers" and those that are non-superimposable mirror images of each other are termed "enantiomers". When a compound has an asymmetric centre, for example, it is bonded to four different groups, a pair of enantiomers is possible. An enantiomer may be characterised by the absolute configuration of its asymmetric centre and is described by the R- and S-sequencing rules of Cahn and Prelog, or by the manner in which the molecule rotates the plane of polarised light and designated as dextrorotatory or laevorotatory (i.e., as (+) or (-)-isomers respectively). A chiral compound may exist as either individual enantiomers or as a mixture thereof. A mixture containing equal proportions of the enantiomers is called a "racemic mixture". Unless otherwise indicated, the description is intended to include individual stereoisomers as well as mixtures. The methods for the determination of stereochemistry and the separation of stereoisomers are well-known in the art (see discussion in Chapter 4 of Advanced Organic Chemistry, 6th edition J. March, John Wiley and Sons, New York, 2007) differ in the chirality of one or more stereocentres.

The present invention also embraces isotopically-labelled compounds which are identical to those recited herein, but for the fact that one or more atoms are replaced by an atom having an atomic mass or mass number different from the atomic mass or mass number usually found in nature. Examples of isotopes that may be incorporated into compounds of the present invention include isotopes of hydrogen, carbon, nitrogen, oxygen, phosphorus, fluorine, and chlorine, such as, but not limited to ²H (deuterium, D), ³H (tritium), ¹¹C, ¹³C, ¹⁴C, ¹⁵N, ¹³F, ³¹P, ³²P, ³⁵S, ³⁶Cl, and ¹²⁵I. Unless otherwise stated, when a position is designated specifically as "H" or "hydrogen", the position is understood to have hydrogen at its natural abundance isotopic composition or its isotopes, such as deuterium (D) or tritium (³H). Certain isotopically-labelled compounds of the present invention (e.g., those labelled with ³H and ¹⁴C) are useful in compound and/or substrate tissue distribution assays. Tritiated (*i.e.*, ³H) and carbon-14 (*i.e.,* ¹⁴C) and fluorine-18 (*i.e.,* ¹⁸F) isotopes are useful for their ease of preparation and detectability. Further, substitution with heavier isotopes such as deuterium (*i.e.,* ²H) may afford certain therapeutic advantages resulting from greater metabolic stability (e.g., increased in vivo half-life or reduced dosage requirements) and hence may be preferred in some circumstances. Isotopically labelled compounds of the present invention may generally be prepared by following procedures analogous to those described herein, by substituting an isotopically labelled reagent for a non-isotopically labelled reagent.

According to the present invention, there is also provided a pharmaceutical composition comprising a compound described herein and a pharmaceutically or therapeutically acceptable excipient or carrier, for example a pharmaceutical composition comprising a compound of general formula (I) and a pharmaceutically or therapeutically acceptable excipient or carrier. For example, a pharmaceutical composition may comprise 4-(6-phenyl-3-pyridyl)-1-azabicyclo[3.2.2]non-3-ene or a pharmaceutically acceptable salt thereof.

Pharmaceutically acceptable excipients or carriers may include fillers, binders, disintegrants, glidants, lubricants, complexing agents, solubilizers, and surfactants, which may be chosen to facilitate administration of the compound by a particular route. Examples of carriers include calcium carbonate, calcium phosphate, various sugars such as lactose, glucose, or sucrose, types of starch, cellulose derivatives, gelatin, lipids, liposomes, nanoparticles, and the like. Carriers also include physiologically compatible liquids as solvents or for suspensions, including, for example, sterile solutions of water for injection (WFI), saline solution, dextrose solution, Hank's solution, Ringer's solution, vegetable oils, mineral oils, animal oils, polyethylene glycols, liquid paraffin, and the like. Excipients may also include, for example, colloidal silicon dioxide, silica gel, talc, magnesium silicate, calcium silicate, sodium aluminosilicate, magnesium trisilicate, powdered cellulose, macrocrystalline cellulose, carboxymethyl cellulose, cross-linked sodium carboxymethylcellulose, sodium benzoate, calcium carbonate, magnesium carbonate, stearic acid, aluminum stearate, calcium stearate, magnesium stearate, zinc stearate, sodium stearyl fumarate, syloid, stearowet C, magnesium oxide, starch, sodium starch glycolate, glyceryl monostearate, glyceryl dibehenate, glyceryl palmitostearate, hydrogenated vegetable oil, hydrogenated cotton seed oil, castor seed oil mineral oil, polyethylene glycol (e.g. PEG 400 or PEG 4000-8000), polyoxyethylene glycol, poloxamers, povidone, crospovidone, croscarmellose sodium, alginic acid, casein, methacrylic acid divinylbenzene copolymer, sodium docusate, cyclodextrins (e.g. 2-hydroxypropyl-.delta.-cyclodextrin), polysorbates (e.g. polysorbate 80), cetrimide, TPGS (d-alpha-tocopheryl polyethylene glycol 1000 succinate), magnesium lauryl sulfate, sodium lauryl sulfate, polyethylene glycol ethers, di-fatty acid ester of polyethylene glycols, or a polyoxyalkylene sorbitan fatty acid ester (e.g., polyoxyethylene sorbitan ester Tween^{®}), polyoxyethylene sorbitan fatty acid esters, sorbitan fatty acid ester, e.g. a sorbitan fatty acid ester from a fatty acid such as oleic, stearic or palmitic acid, mannitol, xylitol, sorbitol, maltose, lactose, lactose monohydrate or lactose spray dried, sucrose, fructose, calcium phosphate, dibasic calcium phosphate, tribasic calcium phosphate, calcium sulfate, dextrates, dextran, dextrin, dextrose, cellulose acetate, maltodextrin, simethicone, polydextrosem, chitosan, gelatin, HPMC (hydroxypropyl methyl celluloses), HPC (hydroxypropyl cellulose), hydroxyethyl cellulose, and the like.

According to the present invention, there is also provided a compound described herein for use in the treatment of a disease or disorder, for example a compound of general formula (I) for use in the treatment of a disease or disorder. For example, 4-(6-phenyl-3-pyridyl)-1-azabicyclo[3.2.2]non-3-ene or a pharmaceutically acceptable salt thereof may be for use in the treatment of a disease or disorder.

According to the present invention, there is also provided a compound described herein for use in the treatment of a disease or disorder mediated by an α7 nicotinic acetylcholine receptor (nAChR), for example a compound of general formula (I) for use in the treatment of a disease or disorder mediated by an α7 nicotinic acetylcholine receptor (nAChR). For example, 4-(3-pyridyl)-1-azabicyclo[3.2.2]non-3-ene or a pharmaceutically acceptable salt thereof may be for use in the treatment of a disease or disorder mediated by an α7 nicotinic acetylcholine receptor (nAChR).

Advantageously, the compounds described herein, for example compounds of general formula (I), competitively inhibit the binding of radiolabeled α-Bungarotoxin to human α7 nAChR subtype with equilibrium constant (Ki) values of 1-1000 nM. It follows that the compounds described herein, for example compounds of general formula (I) are suitable for use in the treatment of a disease or disorder mediated by an α7 nicotinic acetylcholine receptor (nAChR).

The expression "mediated", for example "mediated by nAChR", is understood to mean linked to the activity of nAChR.

For example, the expression "mediated" may be understood to mean "controlled by", for example "transcriptionally or translationally controlled", or "transcriptionally or translationally regulated", for example, up-regulated or down-regulated. Preferably, the expression "mediated by nAChR" may be understood to relate to activation (i.e. opening) of the nAChR ion channel. More specifically, the conformational changes of the nAChR ion channel may occur upon binding a compound leading to a quaternary symmetrical twist of M1-M4 domains that opens the hydrophobic gate and in turn allows the passage of ions. For example, this process may involve permeable to monovalent Na⁺ and K⁺ ions, and Ca²⁺ ions. Advantageously, the ability of nAChRs to alter intracellular calcium levels may lead to activation of different downstream intracellular pathways such as neuronal signalling and plasticity. Moreover, the expression "mediated" may be understood to include to the promotion of intracellular signalling, for example, through a G protein binding cluster contained in the intracellular loop of the α7 subunit.

Optionally, the compounds described herein, for example compounds of general formula (I), may desensitize nAChR. It follows that the compounds described herein, for example compounds of general formula (I), may be suitable for desensitizing nAChR. Accordingly, there is also provided a compound described herein, for example compounds of general formula (I), for use as a nAChR desensitizer.

More specifically, nAChR may rapidly transform to a closed state and followed by a desensitized state following activation. Desensitization is a loss of the biological response due to agonist stimulation. Upon washout of a ligand, the receptor may return to the basal or resting allosteric state.

According to the present invention, there is also provided the use of a compound described herein in the manufacture of a medicament for the treatment of a disease or disorder, for example the use of a compound of general formula (I) in the manufacture of a medicament for the treatment of a disease or disorder.

According to the present invention, there is also provided a compound described herein for use in a method of treating a disease or disorder, comprising the step of administering a compound described herein, or a pharmaceutical composition described herein, to a patient in need of same. For example, there is provided a compound described herein for use in a method of treating a disease or disorder, comprising the step of administering a compound of general formula (I), or a pharmaceutical composition comprising a compound of general formula (I), to a patient in need of same. For example, a compound described herein for use in a method of treating a disease or disorder may comprise the step of administering 4-(6-phenyl-3-pyridyl)-1-azabicyclo[3.2.2]non-3-ene, or a pharmaceutical composition described herein, to a patient in need of same.

Optionally, the disease or disorder is a disease or disorder mediated by an α7 nAChR.

Optionally, the disease or disorder is selected from: a cognitive and neurodegenerative disease; a psychotic disorder, such as schizophrenia; acute nociceptive, neuropathic or inflammatory pain; and an affective disorder, such as depression and inflammation.

For example, the disease or disorder may be selected from the group consisting of:
i) pain, including one or more of acute, neurologic, inflammatory, neuropathic, chronic pain, severe chronic pain, post-operative pain, pain associated with cancer, angina, renal or biliary colic, menstruation, migraine, gout, arthritis, rheumatoid disease, teno-synovitis, vasculitis, trigeminal or herpetic neuralgia, diabetic neuropathy pain, causalgia, low back pain, deafferentation syndromes, and brachial plexus avulsion;
ii) metabolic syndrome, weight gain, type I diabetes mellitus, type II diabetes mellitus, or diabetic neuropathy;
iii) inflammation, including one or more of psoriasis, asthma, atherosclerosis, idiopathic pulmonary fibrosis, chronic and acute inflammation, psoriasis, endotoxemia, gout, acute pseudogout, acute gouty arthritis, arthritis, rheumatoid arthritis, osteoarthritis, allograft rejection, chronic transplant rejection, asthma, atherosclerosis, mononuclear-phagocyte dependent lung injury, atopic dermatitis, chronic obstructive pulmonary disease, adult respiratory distress syndrome, acute chest syndrome in sickle cell disease, inflammatory bowel disease, Crohn's disease, ulcerative colitis, acute cholangitis, aphteous stomatitis, pouchitis, glomerulonephritis, lupus nephritis, thrombosis, and graft vs. host reaction; and/or
iv) cognition, including one or more of age-associated memory impairment, mild cognitive impairment, pre-senile dementia, early onset Alzheimer's disease, senile dementia, dementia of the Alzheimer's type, mild to moderate dementia of the Alzheimer's type, Lewy body dementia, vascular dementia, Alzheimer's disease, stroke, AIDS dementia complex, attention deficit disorder, attention deficit hyperactivity disorder, dyslexia, schizophrenia, schizophreniform disorder, schizoaffective disorder, cognitive deficits in schizophrenia, and cognitive dysfunction in schizophrenia.

The pharmaceutical compositions may be for use in ameliorating any of the symptoms associated with those conditions, diseases and disorders.

Preferably, the treatment is for preventing and/or suppressing cough, the progression of cough, ameliorate symptoms of cough, and ameliorate to the recurrence of cough. The treatment may be for the treatment of cough origins. Hereafter, a "cough" is understood to relate to the expelling of air from the lungs, for example, from the lungs of a subject, for example a human subject. Advantageously, compounds of the present invention, for example compounds of general formula (I), may be used as antitussive agents. Accordingly, there is also provided a compound described herein for use as an antitussive agent, for example a compound of general formula (I) for use as an antitussive agent. More specifically, the antitussive agent may inhibit a cough through a central mechanism, or a peripheral mechanism, or a mixture of the two mechanisms.

The pharmaceutical composition described herein may be used as a selective α7 nAChR agonist. The pharmaceutical composition described herein may also be used as a antitussive agent. The pharmaceutical composition described herein may also be used as an anti-inflammatory agent, for example as an anti-inflammatory agent for acute lung injury, allergen challenge and/or gram-negative bacterial infection. Consequently, pharmaceutical composition described herein may for use in the treatment of inflammation, e.g. in the treatment of an acute lung injury, in the treatment of an allergen challenge and/or in the treatment of gram-negative bacterial infection.

The compounds described herein may also be used in as adjunct therapy in combination with existing therapies, for example, in the management of any the aforementioned types of diseases and disorders. In such situations, it is preferably to administer the active ingredients in a manner that minimizes effects upon nAChR subtypes such as those that are associated with muscle and ganglia. This may be accomplished by targeted drug delivery and/or by adjusting the dosage such that a desired effect is obtained without meeting the threshold dosage required to achieve significant side effects.

Optionally, the compound for use, use or method according described here, may be such that the compound is administered orally.

Optionally, the compound for use, use or method according described here, may be such that the compound is administered in a dry-powder form. Preferably, the compound is dry-powder 4-(6-phenyl-3-pyridyl)-1-azabicyclo[3.2.2]non-3-ene, for example, dry-powder insufflated 4-(6-phenyl-3-pyridyl)-1-azabicyclo[3.2.2]non-3-ene.

Optionally, the compound for use, use or method according described here, may be such that the compound is administered in a nebulised form. Preferably, the compound is nebulised 4-(6-phenyl-3-pyridyl)-1-azabicyclo[3.2.2]non-3-ene.

Optionally, the compound for use, use or method according described here, may be such that the compound is orally administered at a dose of from 0.1 mg to 1 g/kg per day.

For example, the compound may be orally administered at a dose of at least about 0.00001 mg/kg per day, at least about 0.0001 mg/kg per day, at least about 0.001 mg/kg per day, at least about 0.01 mg/kg per day, at least about 0.1 mg/kg per day, at least about 0.2 mg/kg per day, at least about 0.3 mg/kg per day, at least about 0.4 mg/kg per day, at least about 0.5 mg/kg per day. The compound may comprise any range from the given endpoints.

For example, the compound may be the compound is orally administered at a dose of no more than about 0.01 g/kg per day, no more than about 0.1 g/kg per day, no more than about 0.5 g/kg per day, no more than about 0.9 g/kg per day, no more than about 1 g/kg per day, no more than about 1.1 g/kg per day, no more than about 1.2 g/kg per day, no more than about 1.5 g/kg per day, no more than about 2 g/kg per day. The compound may comprise any range from the given endpoints.

For example, the compound may be orally administered at a dose of at least about 0.00001 mg/kg per day to no more than about 2 g/kg per day, at least about 0.0001 mg/kg per day to no more than about 1.5 g/kg per day, at least about 0.001 mg/kg per day to no more than about 1.2 g/kg per day, at least about 0.01 mg/kg per day to no more than about 1.1 g/kg per day, at least about 0.1 mg/kg per day to no more than about 1 g/kg per day, at least about 0.2 mg/kg per day to no more than about 0.9 g/kg per day, at least about 0.3 mg/kg per day to no more than about 0.5 g/kg per day, at least about 0.4 mg/kg per day to no more than about 0.1 g/kg per day, at least about 0.5 mg/kg per day to no more than about 0.01 g/kg per day. The compound may comprise any range from the given endpoints.

For example, the compound for use, use or method according described here, may be such that the compound is orally administered at 0.1 mg/kg per day, 0.2 mg/kg per day, 0.3 mg/kg per day, 0.4 mg/kg per day, 0.5 mg/kg per day, 1 mg/kg per day, 2 mg/kg per day, 3 mg/kg per day, 4 mg/kg per day, 5 mg/kg per day, 10 mg/kg per day, 15 mg/kg per day, 20 mg/kg per day, 25 mg/kg per day, 30 mg/kg per day, 35 mg/kg per day, 40 mg/kg per day, 45 mg/kg per day, 50 mg/kg per day, 100 mg/kg per day, 150 mg/kg per day, 200 mg/kg per day, 250 mg/kg per day, 300 mg/kg per day, 350 mg/kg per day, 400 mg/kg per day, 450 mg/kg per day, 500 mg/kg per day, 550 mg/kg per day, 600 mg/kg per day, 650 mg/kg per day, 700 mg/kg per day, 750 mg/kg per day, 800 mg/kg per day, 850 mg/kg per day, 900 mg/kg per day, 950 mg/kg per day, 1000 mg/kg per day, 1100 mg/kg per day, 1200 mg/kg per day, 1300 mg/kg per day, 1400 mg/kg per day, 1500 mg/kg per day, 1600 mg/kg per day, 1700 mg/kg per day, 1800 mg/kg per day, 1900 mg/kg per day, 2000 mg/kg per day. The compound may comprise any range from the given endpoints.

Optionally, the compound for use, use or method described herein may be such that the compound is orally administered at 5 mg to 2 g/day.

For example, the compound may be orally administered at a dose of at least about 0.1 mg/day, at least about 1 mg/day, at least about 3 mg/day, at least about 4 mg/day, at least about 5 mg/day, at least about 5.1 mg/day, at least about 6 mg/day, at least about 10 mg/day, at least about 50 mg/day. The compound may comprise any range from the given endpoints.

For example, the compound may be orally administered at a dose of no more than about 0.2 g/day, no more than about 0.5 g/day, no more than about 1 g/day, no more than about 1.5 g/day, no more than about 2 g/day, no more than about 2.5 g/day, no more than about 3 g/day, no more than about 5 g/day, no more than about 10 g/day. The compound may comprise any range from the given endpoints.

For example, the compound may be orally administered at a dose of at least about 0.1 mg/day to no more than about 10 g/day, at least about 1 mg/day to no more than about 5 g/day, at least about 3 mg/day to no more than about 3 g/day, at least about 4 mg/day to no more than about 2.5 g/day, at least about 5 mg/day to no more than about 2 g/day, at least about 5.1 mg/day to no more than about 1.5 g/day, at least about 6 mg/day to no more than about 1 g/day, at least about 10 mg/day to no more than about 0.5 g/day, at least about 50 mg/day to no more than about 0.2 g/day. The compound may comprise any range from the given endpoints.

For example, the compound may be orally administered at a dose of 1 mg/day, 2 mg/day, 3 mg/day, 4 mg/day, 5 mg/day, 6 mg/day, 7 mg/day, 8 mg/day, 9 mg/day, 10 mg/day, 11 mg/day, 12 mg/day, 13 mg/day, 14 mg/day, 15 mg/day, 20 mg/day, 25 mg/day, 30 mg/day, 35 mg/day, 40 mg/day, 45 mg/day, 50 mg/day, 100 mg/day, 150 mg/day, 200 mg/day, 250 mg/day, 300 mg/day, 350 mg/day, 400 mg/day, 450 mg/day, 500 mg/day, 550 mg/day, 600 mg/day, 650 mg/day, 700 mg/day, 750 mg/day, 800 mg/day, 850 mg/day, 900 mg/day, 950 mg/day, 1000 mg/day, 1100 mg/day, 1200 mg/day, 1300 mg/day, 1400 mg/day, 1500 mg/day, 1600 mg/day, 1700 mg/day, 1800 mg/day, 1900 mg/day, 2000 mg/day, 2100 mg/day, 2200 mg/day, 2300 mg/day, 2400 mg/day, 2500 mg/day. The compound may comprise any range from the given endpoints.

The expression "about" in relation to numerical values is understood to mean ±10%, preferably ±5%, *e.g.* ±10% w/w, preferably ±5% w/w.

Optionally, the compound for use, use or method described herein may be such that the composition is in the form of a tablet. The tablet may be a film-coated tablet. The compound for use, use or method described herein may be such that the composition is in the form of a capsule, for example a gelatin capsule.

Optionally, the compound for use, use or method described herein may be such that the compound is administered parenterally, topically, rectally, transmucosally, or intestinally.

According to the present invention, there is also provided a method of manufacturing a compound of general formula (I) as shown in Scheme (I):

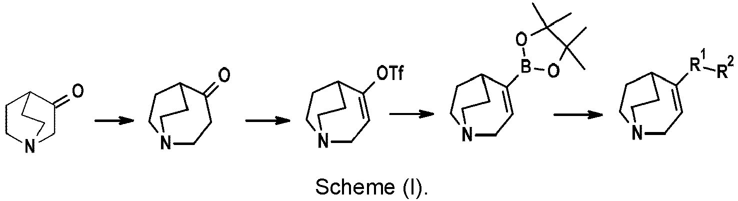

Optionally, R¹ and R² are as defined above, for example, for a compound of general formula (I), R¹ and R² are each an optionally substituted aryl group which may be the same or different, wherein one or both aryl groups comprises a carbocyclic or heterocyclic aromatic ring and wherein one or both aryl groups is monocyclic or polycyclic, and wherein R¹ and R² are optionally fused. Alternatively, for a compound of general formula (I), R¹ is an optionally substituted aryl or an optionally substituted heteroaryl, and R² is a halogen, a triflate, an optionally substituted aryl, an optionally substituted heteroaryl or is absent.

As will be apparent to the skilled person, compounds of formula (I) include 4-substituted 1-azabicyclo[3.2.2]non-3-enes. While the methods by which compounds of the present invention can be prepared may vary, the compounds may be advantageously prepared using intermediates generated during the synthesis of target compounds, which is shown in Scheme (I) above.

While other synthetic strategies will be apparent to those of skilled in the art, aryl substituted 1-azabicyclo[3.2.2]non-3-enes can be made starting from quinuclidin-3-one. The Büchner-Curtius-Schlotterbeck reaction can be used to facilitate one carbon ring expansions of quinuclidin-3-one following triflation and Suzuki coupling with aryl halogenides. Diverse combination of aryl groups in Formula (I) may be achieved via the Suzuki cross-coupling reaction.

### Specific examples of compounds

Examples of representative compounds of the present invention include (but are not limited to) the following compounds:
4-(3-pyridyl)-1-azabicyclo[3.2.2]non-3-ene 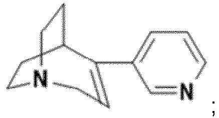
4-(6-phenyl-3-pyridyl)-1-azabicyclo[3.2.2]non-3-ene 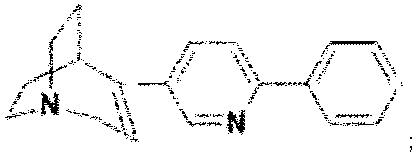 and
4-(6-phenylpyridazin-3-yl)-1-azabicyclo[3.2.2]non-3-ene

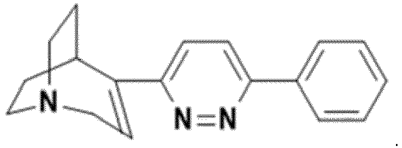

### Numbered Paragraphs

Aspects of the invention are described in the following numbered paragraphs:
1. A compound of general formula (I): 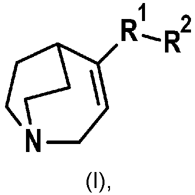 or a pharmaceutically acceptable salt thereof,
   wherein
   R¹ is an optionally substituted aryl or an optionally substituted heteroaryl; and
   R² is a halogen, a triflate, an optionally substituted aryl, an optionally substituted heteroaryl or is absent.
2. The compound according to numbered paragraph 1, wherein the or each aromatic ring of the carbocyclic or heterocyclic aromatic ring of one or both aryl groups is a 3- to 10-membered ring, for example a 5-membered ring or a 6-membered ring.
3. The compound according to numbered paragraph 1 or numbered paragraph 2, wherein one or both aryl groups are monocyclic.
4. The compound according to numbered paragraph 3, wherein one or both aryl groups are independently selected from the group consisting of: phenyl, furanyl, pyrrolyl, thienyl, pyridinyl, pyrimidinyl, pyridazinyl, pyrazinyl, oxazolyl, oxadiazolyl, isoxazolyl, pyrazolyl, imidazolyl, thiazolyl, isothiazolyl and thiadiazolyl.
5. The compound according to either of numbered paragraph 1 or numbered paragraph 2, wherein one or both aryl groups are polycyclic.
6. The compound according to numbered paragraph 5, wherein one or both aryl groups are independently selected from the group consisting of: naphthalenyl, anthracenyl, indolizinyl, indolyl, isoindolyl, benzofuranyl, benzothiophenyl, benzodioxolanyl, indazolyl, pyrrolopyridinyl, benzimidazolyl, benzothiazolyl, benzoisothiazolyl, purinyl, thienopyrazinyl, quinolinyl, isoquinolinyl, cinnolinyl, phthalazinyl, quinazolinyl, quinoxalinyl, 1,8-naphthyridinyl, pteridinyl, carbazolyl, acridinyl, naphtiridinyl, phenazinyl, phenothiazinyl, phenoxazinyl and azulenyl.
7. The compound according to any preceding numbered paragraph, wherein one or both aryl groups are substituted, for example with 1, 2 or 3 substituents.
8. The compound according to numbered paragraph 7, wherein the substituents are independently selected from the group consisting of: alkyl, alkenyl, heterocyclyl, cycloalkyl, aryl, substituted aryl, arylalkyl, substituted arylalkyl, halo (for example, F, Cl, Br or I), --OR', --NR'R", --CF3, --CN, --NO₂, --SR', --N₃, --C(=O)NR'R", --NR'C(=O)R", --C(=O)R', --C(=O)OR', --OC(=O)R',-O(CR'R"), --C(=O)R', --SO₂R', and --SO₂NR'R", wherein R' and R" are individually hydrogen, lower alkyl (for example, straight chain or branched alkyl including C₁-C₈, preferably C₁-C₅, such as methyl, ethyl or isopropyl), cycloalkyl, heterocyclyl, aryl, or arylalkyl (such as benzyl), and wherein R' and R" optionally combine to form a cyclic functionality.
9. The compound according to numbered paragraph 8, wherein the substituents are independently selected from the group consisting of: cyano, halo, alkyl, haloalkyl, cycloalkyl, alkoxy, haloalkoxy and alkylthio.
10. The compound according to any preceding numbered paragraph, wherein R¹ is selected from: 1,3,4-thiadiazolyl, pyridinyl, pyridazinyl, pyrimidinyl and pyrazinyl.
11. The compound according to any preceding numbered paragraph, wherein R² is selected from indolyl, benzofuranyl, benzothiazolyl and phenyl.
12. The compound according to any preceding numbered paragraph, wherein the compound is selected from the group consisting of:
   4-(3-pyridyl)-1-azabicyclo[3.2.2]non-3-ene;
   4-(6-phenyl-3-pyridyl)-1-azabicyclo[3.2.2]non-3-ene; and
   4-(6-phenylpyridazin-3-yl)-1-azabicyclo[3.2.2]non-3-ene.
13. The compound according to numbered paragraph 12, wherein the compound is 4-(6-phenyl-3-pyridyl)-1-azabicyclo[3.2.2]non-3-ene.
14. The compound according to any preceding numbered paragraph, wherein the pharmaceutically acceptable salt is selected from one or more of the following: chloride, bromide, sulfate, phosphate, and nitrate; organic acid addition salts such as acetate, galactarate, propionate, succinate, lactate, glycolate, malate, tartrate, citrate, maleate, fumarate, methanesulfonate, p-toluenesulfonate, ascorbate; salts with acidic amino acids, for example aspartate or glutamate, or hydrates or ethanol solvates.
15. A pharmaceutical composition comprising a compound according to any of numbered paragraphs 1 to 14 and a pharmaceutically or therapeutically acceptable excipient or carrier.
16. A compound according to any of numbered paragraphs 1 to 14 for use in the treatment of a disease or disorder.
17. A compound according to any of numbered paragraphs 1 to 14 for use in the treatment for use in the treatment of a disease or disorder mediated by an α7 nicotinic acetylcholine receptor (nAChR).
18. Use of a compound according to any of numbered paragraphs 1 to 14 in the manufacture of a medicament for the treatment of a disease or disorder.
19. The compound for use according to numbered paragraph 16 or numbered paragraph 17, or the use according to numbered paragraph 18, wherein the treatment is for preventing and/or suppressing cough, the progression of cough, ameliorate symptoms of cough, and ameliorate to the recurrence of cough.
20. The compound for use according to numbered paragraph 16 or numbered paragraph 17, or the use according to numbered paragraph 18, wherein the disease or disorder is selected from: a cognitive and neurodegenerative disease; a psychotic disorder, such as schizophrenia; acute nociceptive, neuropathic or inflammatory pain; and an affective disorder, such as depression and inflammation.
21. The compound for use, or use according to numbered paragraph 19 or numbered paragraph 20, wherein the compound is administered orally.
22. The compound for use, or use according to numbered paragraph 21, wherein the compound is orally administered at a dose of from 0.1 mg to 1 g/kg per day.
23. The compound for use, or use according to numbered paragraph 21, wherein the compound is orally administered at a dose of at least about 0.00001 mg/kg per day, at least about 0.0001 mg/kg per day, at least about 0.001 mg/kg per day, at least about 0.01 mg/kg per day, at least about 0.1 mg/kg per day, at least about 0.2 mg/kg per day, at least about 0.3 mg/kg per day, at least about 0.4 mg/kg per day, at least about 0.5 mg/kg per day.
24. The compound for use, or use according to numbered paragraph 21, wherein the compound is orally administered at a dose of no more than about 0.01 g/kg per day, no more than about 0.1 g/kg per day, no more than about 0.5 g/kg per day, no more than about 0.9 g/kg per day, no more than about 1 g/kg per day, no more than about 1.1 g/kg per day, no more than about 1.2 g/kg per day, no more than about 1.5 g/kg per day, no more than about 2 g/kg per day.
25. The compound for use, or use according to numbered paragraph 21, wherein the compound is orally administered at a dose of at least about 0.00001 mg/kg per day to no more than about 2 g/kg per day, at least about 0.0001 mg/kg per day to no more than about 1.5 g/kg per day, at least about 0.001 mg/kg per day to no more than about 1.2 g/kg per day, at least about 0.01 mg/kg per day to no more than about 1.1 g/kg per day, at least about 0.1 mg/kg per day to no more than about 1 g/kg per day, at least about 0.2 mg/kg per day to no more than about 0.9 g/kg per day, at least about 0.3 mg/kg per day to no more than about 0.5 g/kg per day, at least about 0.4 mg/kg per day to no more than about 0.1 g/kg per day, at least about 0.5 mg/kg per day to no more than about 0.01 g/kg per day.
26. The compound for use, or use according to numbered paragraph 21, wherein the compound is orally administered at 5 mg to 2 g/day.
27. The compound for use, or use according to numbered paragraph 21, wherein the compound is orally administered at a dose of at least about 0.1 mg/day, at least about 1 mg/day, at least about 3 mg/day, at least about 4 mg/day, at least about 5 mg/day, at least about 5.1 mg/day, at least about 6 mg/day, at least about 10 mg/day, at least about 50 mg/day.
28. The compound for use, or use according to numbered paragraph 21, wherein the compound is orally administered at a dose of no more than about 0.2 g/day, no more than about 0.5 g/day, no more than about 1 g/day, no more than about 1.5 g/day, no more than about 2 g/day, no more than about 2.5 g/day, no more than about 3 g/day, no more than about 5 g/day, no more than about 10 g/day.
29. The compound for use, or use according to numbered paragraph 21, wherein the compound is orally administered at a dose of at least about 0.1 mg/day to no more than about 10 g/day, at least about 1 mg/day to no more than about 5 g/day, at least about 3 mg/day to no more than about 3 g/day, at least about 4 mg/day to no more than about 2.5 g/day, at least about 5 mg/day to no more than about 2 g/day, at least about 5.1 mg/day to no more than about 1.5 g/day, at least about 6 mg/day to no more than about 1 g/day, at least about 10 mg/day to no more than about 0.5 g/day, at least about 50 mg/day to no more than about 0.2 g/day.
30. The compound for use, or use according to any of numbered paragraphs 21 to 29, wherein the composition is in the form of a tablet.
31. The compound for use, or use according to numbered paragraph 19 or numbered paragraph 20, wherein the compound is administered parenterally, topically, rectally, transmucosally, or intestinally.
32. A method of manufacturing a compound of general formula (I) as shown in Scheme (I): 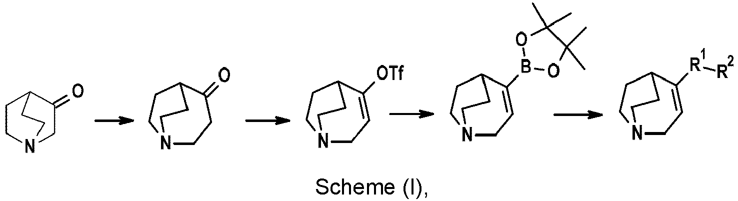 wherein R¹ and R² are as defined in any of numbered paragraphs 1 to 11.

### List of Figures

**Figure 1** is a flow chart showing the evaluation of cough responses in a cough model.
**Figure 2** is an arrangement of bar charts showing the effect of cough of 4-(6-phenyl-3-pyridyl)-1-azabicyclo[3.2.2]non-3-ene on cumulative number of citric acid-induced coughs.
**Figure 3** is an arrangement of bar charts showing the effect of 4-(6-phenyl-3-pyridyl)-1-azabicyclo[3.2.2]non-3-ene on cough incubation period of citric acid-induced coughs.

### Experimental

Synthetic examples of compounds set out in Examples 1-5, and the biological activity examples in Example 6 and 7 are provided to illustrate the present invention and should not be construed as limiting the scope thereof. In these examples, all parts and percentages are by weight, unless otherwise noted. Reaction yields are reported in mole percentage.

### Example 1 (Reference): 1-Azabicyclof3.2.21nonan-4-one (cas: 30708-54-4)

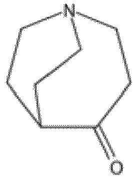

To a solution of trimethylsilyldiazomethane (2.00 M, 95.8 mL, 1.20 eq) was added quinuclidin-3-one (20.0 g, 159 mmol, 1.00 eq) in tetrahydrofuran (120 mL) at 5 °C. And then methanol (70 mL) was added, the mixture was stirred at 25 °C for 12 hrs. TLC (Dichloromethane/Methanol = 10/1) showed one major spot (Rf = 0.35) was detected. After addition of acetic acid (100 drops), the mixture was poured into the solution of sodium carbonate (100 mL), and the organic layer was separated. The aqueous layer was extracted with dichloromethane (200 mL * 3). The combined organic layers were dried over Na2SO4, filtered and concentrated under reduced pressure. 1-Azabicyclo[3.2.2]nonan-4-one (20.0 g, crude) was obtained as yellow oil. 1H NMR (400 MHz, CDCl3): δ 3.14-3.01 (m, 2H), 2.99-2.90 (m, 4H), 2.67-2.66 (m, 2H), 2.64-2.53 (m, 1H), 1.80-1.77 (m, 2H), 1.72-1.68 (m, 2H).

### Example 2: 1-Azabicyclo[3.2.2]non-3-en-4-yl trifluoromethanesulfonate

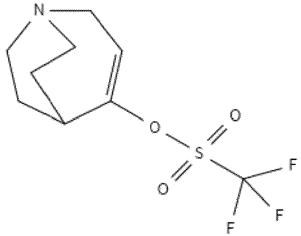

To a solution of lithium dimethylamide (2.00 M, 23.3 mL, 1.30 eq) in tetrahydrofuran (10mL) was added a solution of 1-azabicyclo[3.2.2]nonan-4-one (5.00 g, 35.9 mmol, 1.00 eq) in tetrahydrofuran (30 mL) at -78 °C and stirred at -78 °C for 30 min. Then to the mixture was added a solution of bis(trifluoromethanesulfonyl)aniline (16.6 g, 46.7 mmol, 1.30 eq) in tetrahydrofuran (5mL). The reaction mixture was quenched by addition of saturated NaHCO3 (10 mL) at 25 °C, and then filtered and concentrated under reduced pressure. The residue was purified by column chromatography (SiO2, dichloromethane/methanol = 1/0 to 10/1). 1-Azabicyclo[3.2.2]non-3-en-4-yl trifluoromethanesulfonate (10.0 g, crude) was obtained as a yellow oil. 1H NMR (400 MHz, CDCl3): δ 5.65-5.63 (m, 1H), 3.62 (d, J=2.9 Hz, 2H), 3.15-3.05 (m, 4H), 2.48-2.47 (m, 1H), 2.24-2.21 (m, 2H), 1.88-1.84 (m, 2H).

### Example 3: 4-(3-Pyridyl)-1-azabicyclof3.2.21non-3-ene

A solution of 1-azabicyclo[3.2.2]non-3-en-4-yl trifluoromethanesulfonate (1.00 g, 3.69 mmol, 1.00 eq), pyridine-3-boronic acid (679 mg, 5.53 mmol, 1.50 eq), K2CO3 (1.53 g, 11.0 mmol, 3.00 eq), Pd(dppf)Cl2 (134 mg, 184 µmol, 0.0500 eq) in water (2 mL) and dioxane (10 mL) was stirred at 90 °C for 3 hrs. The reaction mixture was filtered and concentrated under reduced pressure. The residue was purified by column chromatography (SiO2, dichloromethane/methanol =1/0 to 5/1; (1%NH3•H2O)) and by prep-HPLC (basic condition; column: Waters Xbridge 150*25mm* 5um; mobile phase: [water (0.05% ammonia hydroxide v/v)-ACN]; B%: 17%-45%, 10min). 4-(3-Pyridyl)-1-azabicyclo[3.2.2]non-3-ene (16.0 mg, 79.62 µmol, 2.16% yield, 99.67% purity) was obtained as a light yellow gum. 1H NMR (400 MHz, CD3OD): δ 8.49 (d, J=2.0 Hz, 1H), 8.38 (dd, J=1.5, 4.8 Hz, 1H), 7.78 (td, J=1.9, 8.0 Hz, 1H), 7.37 (dd, J=4.9, 7.9 Hz, 1H), 5.86 (dt, J=1.5, 3.1 Hz, 1H), 3.75 (d, J=3.1 Hz, 2H), 3.13-3.07 (m, 4H), 2.79-2.78 (m, 1H), 2.18-2.13

(m, 2H), 2.06-2.02 (m, 2H). LCMS: m/z = 201 (M+1)+.

### Example 4: 4-(6-Phenyl-3-pyridyl)-1-azabicyclo[3.2.2]non-3-ene

To a solution of 1-azabicyclo[3.2.2]non-3-en-4-yl trifluoromethanesulfonate (500 mg, 1.84 mmol, 1.00 eq) and 2-phenyl-5-(4,4,5,5-tetramethyl-1,3,2-dioxaborolan-2-yl)pyridine (570 mg, 2.03 mmol, 1.10 eq) in water (2 mL) and dioxane (10 mL) was added K2CO3 (764 mg, 5.53 mmol, 3.00 eq) and Pd(dppf)Cl2 (67.4 mg, 92.1 µmol, 0.0500 eq). The mixture was stirred at 90 °C for 10 hrs. The reaction mixture was filtered and concentrated under reduced pressure. The residue was purified by column chromatography (SiO2, dichloromethane/methanol = 1/0 to 10/1). 4-(6-Phenyl-3-pyridyl)-1-azabicyclo[3.2.2]non-3-ene (150 mg, 529 umol, 28.7% yield, 97.1% purity) was obtained as a brown solid. 1H NMR (400 MHz, CD3OD): δ 8.59 (s, 1H), 7.93- 7.82 (m, 3H), 7.50-7.43 (m, 3H), 7.16-6.89 (m, 1H), 5.92 (s, 1H), 3.87 (d, J=3.2 Hz, 2H), 3.26-3.20 (m, 4H), 2.91 (s, 1H), 2.26-2.09 (m, 4H). LCMS: m/z = 277 (M+1)+.

### Example 5: 4-(6-Phenylpyridazin-3-yl)-1-azabicyclo[3.2.2]non-3-ene

STEP1: To a solution of 1-azabicyclo[3.2.2]non-3-en-4-yl trifluoromethanesulfonate (0.500 g, 1.84 mmol, 1.00 eq) and bis(pinacolato)diboron (467 mg, 1.84 mmol, 1.00 eq) in dioxane (10 mL) was added potassium acetate (361 mg, 3.68 mmol, 2.00 eq) and Pd(dppf)Cl2 (67.3 mg, 92.0 µmol, 0.0500 eq). The mixture was stirred at 80 °C for 12 hrs. 3-Chloro-6-phenylpyridazine (420 mg, 2.21 mmol, 1.20 eq), K2CO3 (762 mg, 5.52 mmol, 3.00 eq) and Pd(dppf)Cl2 (67.3 mg, 92.0 µmol, 0.0500 eq) was added into the mixture, the mixture was stirred at 90 °C for 3 hrs. The reaction mixture was filtered and concentrated under reduced pressure. The residue was purified by column chromatography (SiO2, dichloromethane/methanol =1/0 to 10/1). 4-(6-Phenylpyridazin-3-yl)-1-azabicyclo[3.2.2]non-3-ene (50.8 mg, 177 µmol, 9.63% yield, 96.7% purity) was obtained as a grey solid. 1H NMR (400 MHz, CD3OD): δ 8.11-8.07 (m, 2H), 7.93 (d, J=9.0 Hz, 1H), 7.57-7.54 (m, 2H), 7.18-6.93 (m, 2H), 6.43-6.42 (m, 1H), 4.03 (d, J=3.3 Hz, 2H), 3.65-3.64 (m, 1H), 3.32-3.29 (m, 2H), 3.28-3.26 (m, 2H), 2.25-2.09 (m, 4H). LCMS: m/z = 278 (M+1)+.

### Example 6: Biological Activity

Compounds described in Examples 3 to 5 competitively inhibited the binding of radiolabelled α-Bungarotoxin to human α7 nAChR subtype with equilibrium constant (Ki) values of 1-1000 nM.

Human recombinant nicotinic acetylcholine α7 receptor were expressed in SH-SY5Y cells. A 20 µg aliquot of membranes are incubated in modified phosphate buffer pH 7.4 with 0.4 nM [1251]α-Bungarotoxin for 120 minutes at 37° C. Non-specific binding is estimated in the presence of 1 µM α-Bungarotoxin. Membranes are filtered and washed, and the filters are then counted to determine specifically-bound [1251]α-Bungarotoxin. IC₅₀ values were determined by a non-linear, least squares regression analysis using MathlQTM (ID Business Solutions Ltd., UK). The Ki values were calculated using the equation of Cheng and Prusoff using the observed IC₅₀ of the tested compound, the concentration of radioligand employed in the assay, and the historical values for the KD of the ligand (0.40 nM [1251] α-Bungarotoxin). The Ki values were shown in nM for each compound tested in **Table 1** below.

**Table 1**

| α7 Ki [nM] | Compound |
|---|---|
| 130 | 4-(6-phenyl-3-pyridyl)-1-azabicyclo[3.2.2]non-3-ene |
| 320 | 4-(6-phenylpyridazin-3-yl)-1-azabicyclo[3.2.2]non-3-ene |

### Example 7: In vivo cough induction experiments

### Materials and Methods

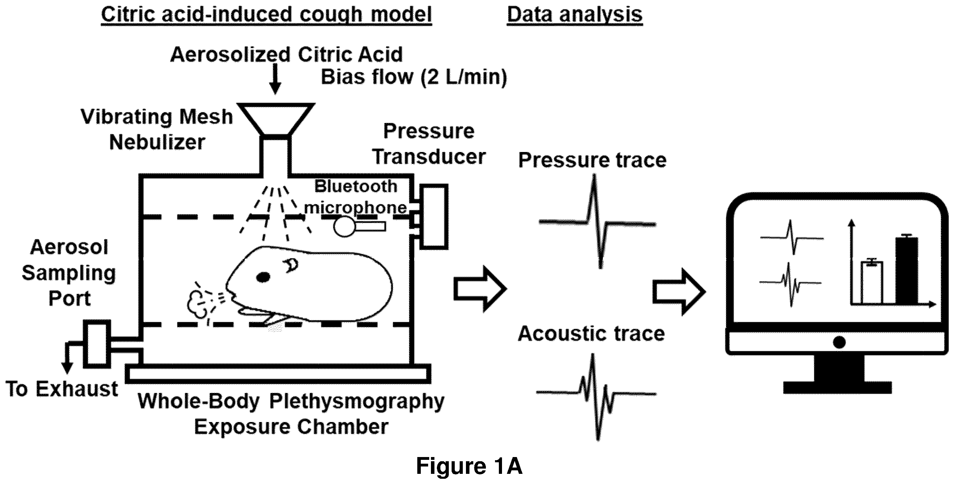

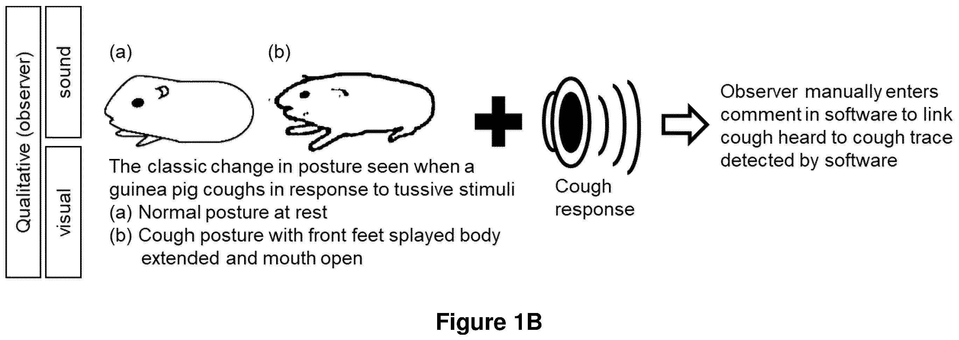

As depicted in **Figure 1****,** cough induction is conducted by placing guinea pigs into a whole body plethysmograph (WBP) equipped with a Aeroneb^{™} vibrating mesh nebulizer, and including pressure transducers and detection equipment (EMKA technologies). A primary microphone is supplied (EMKA Technologies) for acoustic confirmation of cough frequency in the form of a digital trace using the EMKA software. The data acquisition software (iOX2, version 2.10.5.28, EMKA Technologies, France) displays the pressure signal detected from the pressure transducer and calculates both frequency and intensity of coughs measured during the testing period.

A secondary microphone (T1 True Wireless Earphone, QCY^{®}) also is placed in the chamber and connected to a smartphone via Bluetooth to enable the observers to hear the audible sounds during the test. A bias flow rate of 2 L/min is used to continuously draw either atmospheric air (during the fresh air period) or aerosolized citric acid from the nebulizer into the WBP and expel it out through an exhaust pipe. Chamber pressure in the WBP is set to be between -10 to -30 Pa relative to ambient pressure.

Prior to the citric acid challenge, the guinea pigs are administered (intraperitoneal injection) with vehicle control, positive control or test compound (where applicable) with different dosage **(Table 2)** and placed individually into the WBP for at least 5 minutes in order to habituate.

**Table 2**

| | Low | Medium | High |
|---|---|---|---|
| Saline | / | / | / |
| Codeine | 30 mg/kg | 30 mg/kg | 30 mg/kg |
| 4-(6-phenyl-3-pyridyl)-1-azabicyclo[3.2.2]non-3-ene | 1.5 mg/kg | 5 mg/kg | 15 mg/kg |

The procedure was as follows: Injection → mice in box having citric acid for 10 mins (counting the cough) → box then fill with air for 5 mins (counting the cough) → mice move to recovery cage.

Appropriate concentration of citric acid is used to generate aerosol and deliver to WBP for 10 minutes with constant recording of pressure measurements. The mean concentration of citric acid was 1283.1± 137.2 µg/L. Upon completion of citric acid challenge, the guinea pig is allowed to recover and continue recording of cough counts for another 5 minutes. Subsequently, the guinea pigs are closely monitored in recovery cage for full recovery from the challenge and that no adverse events were present before returning the animals to its home cage. Cough counts (CCnt) are recorded over a period of at least 10 mins, starting from citric acid stimulation and cough incubation period (CIP) are defined as the time it takes to cough for the first time after citric acid stimulation.

The cough response during citric acid challenge and post-treatment recovery is carefully monitored using:
- continuous pressure monitoring (plethysmography monitoring software)
- acoustic confirmation of cough using microphones (primary and secondary)
- visual confirmation of cough through posture change.

### Statistical Analysis and Data Representation

The data were analysed using Oneway ANOVA (* p < 0.05, ** p < 0.01, *** p < 0.001, **** p < 0.0001) when compared to saline control, and each column represents Mean ± SEM.

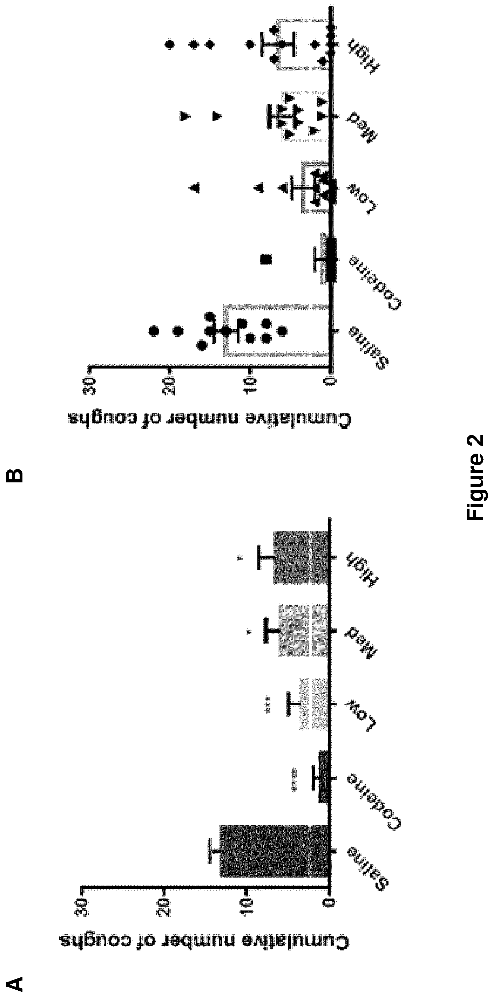

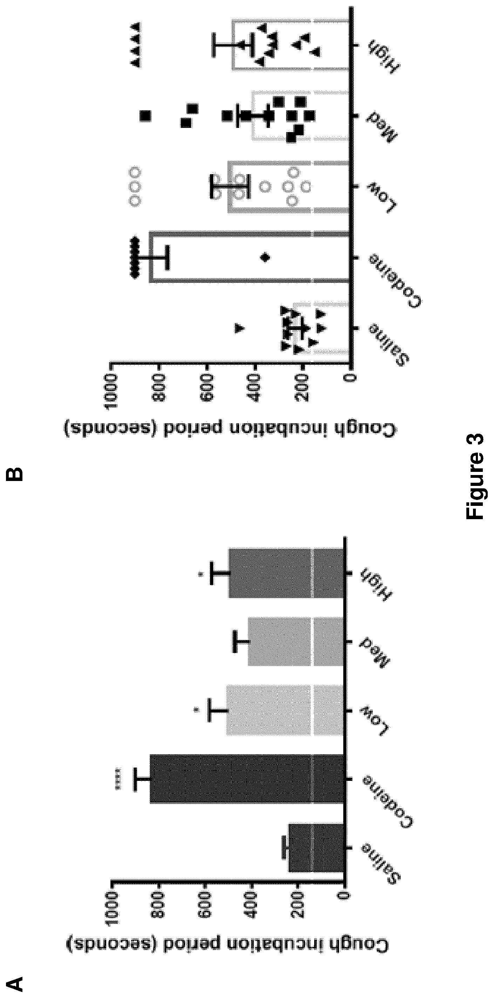

Figures 2 and 3 relate to the assessment of the antitussive efficacy of 4-(6-phenyl-3-pyridyl)-1-azabicyclo[3.2.2]non-3-ene using an *in vivo* model of chemically induced coughs in guinea pigs.

Figures 2A and 3A show the mean ± SEM, whereas Figure 2B and 3B show the results for each replicate, respectively.

### Results

As shown in **Figure 2** relating to cough counts, an example compound of the invention, 4-(6-phenyl-3-pyridyl)-1-azabicyclo[3.2.2]non-3-ene, significantly reduced the citric acid-induced cough counts when compared with saline control.

More specifically, 4-(6-phenyl-3-pyridyl)-1-azabicyclo[3.2.2]non-3-ene significantly reduced citric acid-induced cough counts in low concentration (1.5 mg/kg, number of coughs = 3 , p < 0.0001), medium concentration (5 mg/kg, number of coughs = 6, p < 0.05), high concentration (15 mg/kg, number of coughs = 7, p < 0.05) compared with saline control (0.9% Sodium Chloride, number of coughs = 13).

The reduction of citric acid-induced cough counts by 4-(6-phenyl-3-pyridyl)-1-azabicyclo[3.2.2]non-3-ene was similar to reduction by 30 mg/kg codeine. However, at 1.5 mg/kg to 15 mg/kg, the dose of 4-(6-phenyl-3-pyridyl)-1-azabicyclo[3.2.2]non-3-ene was approximately 20 to 30-fold lower compared with the 30 mg/kg dose of codeine.

Further, as shown in **Figure 3** relating to incubation period, 4-(6-phenyl-3-pyridyl)-1-azabicyclo[3.2.2]non-3-ene showed a significant improvement in cough incubation period.

More specifically, 4-(6-phenyl-3-pyridyl)-1-azabicyclo[3.2.2]non-3-ene significantly increased the cough incubation period in low concentration (1.5 mg/kg, 504 seconds, p < 0.05), high concentration (15 mg/kg, 491 seconds, p < 0.05) compared with saline control (0.9% Sodium Chloride, 232 seconds).

The increase of cough incubation period by 4-(6-phenyl-3-pyridyl)-1-azabicyclo[3.2.2]non-3-ene was similar to that by 30 mg/kg codeine. However, at 1.5 mg/kg to 15 mg/kg, the dose of 4-(6-phenyl-3-pyridyl)-1-azabicyclo[3.2.2]non-3-ene was approximately 20 to 30-fold lower compared with the 30 mg/kg dose of codeine.

### Conclusions

4-(6-phenyl-3-pyridyl)-1-azabicyclo[3.2.2]non-3-ene, an example compound of the invention, significantly reduced citric acid-induced cough counts when compared with saline control (see **Figure 2****).**

Further, 4-(6-phenyl-3-pyridyl)-1-azabicyclo[3.2.2]non-3-ene also showed a significant improvement in cough incubation period at dosage approximately 20 to 30-fold lower than codeine which is considered to be the "gold standard" narcotic cough suppressant (see **Figure 3****).**

## Claims

1. A compound of general formula (I): 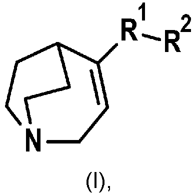 or a pharmaceutically acceptable salt thereof,
wherein
R¹ is an optionally substituted aryl or an optionally substituted heteroaryl; and
R² is a halogen, a triflate, an optionally substituted aryl, an optionally substituted heteroaryl or is absent.

2. The compound according to claim 1, wherein the or each aryl or heteroaryl is a 5- to 10-membered ring, preferably a 5-membered ring or a 6-membered ring.

3. The compound according to claim 1 or claim 2, wherein the or each aryl or heteroaryl groups are monocyclic.

4. The compound according to claim 3, wherein the or each aryl or heteroaryl groups are independently selected from the group consisting of: phenyl, furanyl, pyrrolyl, thienyl, pyridinyl, pyrimidinyl, pyridazinyl, pyrazinyl, oxazolyl, oxadiazolyl, isoxazolyl, pyrazolyl, imidazolyl, thiazolyl, isothiazolyl and thiadiazolyl.

5. The compound according to either of claim 1 or claim 2, wherein the or each aryl or heteroaryl groups are polycyclic.

6. The compound according to claim 5, wherein the or each aryl or heteroaryl groups are independently selected from the group consisting of: naphthalenyl, anthracenyl, indolizinyl, indolyl, isoindolyl, benzofuranyl, benzothiophenyl, benzodioxolanyl, indazolyl, pyrrolopyridinyl, benzimidazolyl, benzothiazolyl, benzoisothiazolyl, purinyl, thienopyrazinyl, quinolinyl, isoquinolinyl, cinnolinyl, phthalazinyl, quinazolinyl, quinoxalinyl, 1,8-naphthyridinyl, pteridinyl, carbazolyl, acridinyl, naphtiridinyl, phenazinyl, phenothiazinyl, phenoxazinyl and azulenyl.

7. The compound according to any preceding claim, wherein the or each aryl or heteroaryl groups are substituted, preferably with 1, 2 or 3 substituents, wherein optionally the substituents are independently selected from the group consisting of: alkyl, alkenyl, heterocyclyl, cycloalkyl, aryl, substituted aryl, arylalkyl, substituted arylalkyl, halo (preferably, F, Cl, Br or I), --OR', --NR'R", --CF3, --CN, --NO₂, --SR', --N₃, --C(=O)NR'R", --NR'C(=O)R", --C(=O)R', --C(=O)OR', --OC(=O)R',-O(CR'R"), --C(=O)R', --SO₂R', and --SO₂NR'R", wherein R' and R" are individually hydrogen, lower alkyl (preferably, straight chain or branched alkyl including C₁-C₈, preferably C₁-C₅, such as methyl, ethyl or isopropyl), cycloalkyl, heterocyclyl, aryl, or arylalkyl (such as benzyl), and wherein R' and R" optionally combine to form a cyclic functionality.

8. The compound according to claim 7, wherein the substituents are independently selected from the group consisting of: cyano, halo, alkyl, haloalkyl, cycloalkyl, alkoxy, haloalkoxy and alkylthio.

9. The compound according to any preceding claim, wherein R¹ is selected from: 1,3,4-thiadiazolyl, pyridinyl, pyridazinyl, pyrimidinyl and pyrazinyl, and/or wherein R² is selected from indolyl, benzofuranyl, benzothiazolyl and phenyl.

10. The compound according to any preceding claim, wherein the compound is selected from the group consisting of:
4-(3-pyridyl)-1-azabicyclo[3.2.2]non-3-ene;
4-(6-phenyl-3-pyridyl)-1-azabicyclo[3.2.2]non-3-ene; and
4-(6-phenylpyridazin-3-yl)-1-azabicyclo[3.2.2]non-3-ene,
preferably wherein the compound is 4-(6-phenyl-3-pyridyl)-1-azabicyclo[3.2.2]non-3-ene.

11. A pharmaceutical composition comprising a compound according to any of claims 1 to 10 and a pharmaceutically or therapeutically acceptable excipient or carrier.

12. A compound according to any of claims 1 to 10 for use in the treatment of a disease or disorder.

13. A compound according to any of claims 1 to 10 for use in the treatment for use in the treatment of a disease or disorder mediated by an α7 nicotinic acetylcholine receptor (nAChR).

14. The compound for use according to claim 12 or claim 13, wherein the treatment is for preventing and/or suppressing cough, the progression of cough, ameliorate symptoms of cough, and ameliorate to the recurrence of cough.

15. A method of manufacturing a compound of general formula (I) as shown in Scheme (I): 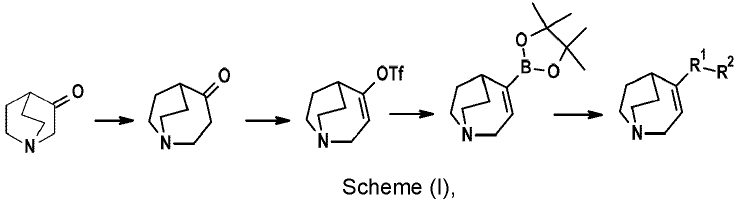 wherein R¹ and R² are as defined in any of claims 1 to 14.

## Patentansprüche

1. Verbindung der allgemeinen Formel (I): 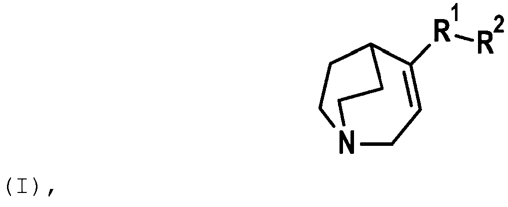
oder ein pharmazeutisch annehmbares Salz davon,
wobei
R¹ ein optional substituiertes Aryl oder ein optional substituiertes Heteroaryl ist; und
R² ein Halogen, ein Triflat, ein optional substituiertes Aryl, ein optional substituiertes Heteroaryl ist oder fehlt.

2. Verbindung nach Anspruch 1, wobei das oder jedes Aryl oder Heteroaryl ein 5- bis 10-gliedriger Ring, bevorzugt ein 5-gliedriger Ring oder ein 6-gliedriger Ring ist.

3. Verbindung nach Anspruch 1 oder Anspruch 2, wobei die oder jede Aryl- oder Heteroarylgruppe monozyklisch ist.

4. Verbindung nach Anspruch 3, wobei die oder jede Aryl- oder Heteroarylgruppe unabhängig voneinander ausgewählt ist aus der Gruppe bestehend aus: Phenyl, Furanyl, Pyrrolyl, Thienyl, Pyridinyl, Pyrimidinyl, Pyridazinyl, Pyrazinyl, Oxazolyl, Oxadiazolyl, Isoxazolyl, Pyrazolyl, Imidazolyl, Thiazolyl, Isothiazolyl und Thiadiazolyl.

5. Verbindung nach Anspruch 1 oder Anspruch 2, wobei die oder jede Aryl- oder Heteroarylgruppe polyzyklisch ist.

6. Verbindung nach Anspruch 5, wobei die oder jede Aryl- oder Heteroarylgruppe unabhängig voneinander ausgewählt ist aus der Gruppe bestehend aus: Naphthalenyl, Anthracenyl, Indolizinyl, Indolyl, Isoindolyl, Benzofuranyl, Benzothiophenyl, Benzodioxolanyl, Indazolyl, Pyrrolopyridinyl, Benzimidazolyl, Benzothiazolyl, Benzoisothiazolyl, Purinyl, Thienopyrazinyl, Chinolinyl, Isochinolinyl, Cinnolinyl, Phthalazinyl, Chinazolinyl, Chinoxalinyl, 1,8-Naphthyridinyl, Pteridinyl, Carbazolyl, Acridinyl, Naphthyridinyl, Phenazinyl, Phenothiazinyl, Phenoxazinyl und Azulenyl.

7. Verbindung nach einem beliebigen vorhergehenden Anspruch, wobei die oder jede Aryl- oder Heteroarylgruppe substituiert ist, bevorzugt mit 1, 2 oder 3 Substituenten, wobei die Substituenten optional unabhängig voneinander ausgewählt sind aus der Gruppe bestehend aus: Alkyl, Alkenyl, Heterocyclyl, Cycloalkyl, Aryl, substituiertem Aryl, Arylalkyl, substituiertem Arylalkyl, Halogen (bevorzugt F, Cl, Br oder I), --OR', --NR'R',, --CF3, --CN, --NO₂, --SR', --N₃, --C(=O)NR'R", --NR'C(=O)R',, --C(=O)R', --C(=O)OR', --OC(=O)R', --O(CR'R"), --C(=O)R', --SO₂R' und --SO₂NR'R',, wobei R' und R" einzeln Wasserstoff, Niederalkyl (bevorzugt geradkettiges oder verzweigtes Alkyl, einschließlich C₁-C₈, bevorzugt C₁-C₅, wie Methyl, Ethyl oder Isopropyl), Cycloalkyl, Heterocyclyl, Aryl oder Arylalkyl (wie Benzyl) sind, und wobei R' und R'' sich optional kombinieren, um eine zyklische Funktionalität zu bilden.

8. Verbindung nach Anspruch 7, wobei die Substituenten unabhängig voneinander ausgewählt sind aus der Gruppe bestehend aus: Cyano, Halogen, Alkyl, Halogenalkyl, Cycloalkyl, Alkoxy, Halogenalkoxy und Alkylthio.

9. Verbindung nach einem beliebigen vorhergehenden Anspruch, wobei R¹ ausgewählt ist aus: 1,3,4-Thiadiazolyl, Pyridinyl, Pyridazinyl, Pyrimidinyl und Pyrazinyl, und/oder wobei R² ausgewählt ist aus Indolyl, Benzofuranyl, Benzothiazolyl und Phenyl.

10. Verbindung nach einem beliebigen vorhergehenden Anspruch, wobei die Verbindung ausgewählt ist aus der Gruppe bestehend aus:
4-(3-Pyridyl)-1-azabicyclo[3.2.2]non-3-en;
4-(6-Phenyl-3-pyridyl)-1-azabicyclo[3.2.2]non-3-en; und
4-(6-Phenylpyridazin-3-yl)-1-azabicyclo[3.2.2]non-3-en,
bevorzugt wobei die Verbindung 4-(6-Phenyl-3-pyridyl)-1-azabicyclo[3.2.2]non-3-en ist.

11. Pharmazeutische Zusammensetzung umfassend eine Verbindung nach einem der Ansprüche 1 bis 10 und einen pharmazeutisch annehmbaren oder therapeutisch akzeptablen Trägerhilfsstoff oder Träger.

12. Verbindung nach einem der Ansprüche 1 bis 10 zum Gebrauch bei der Behandlung einer Krankheit oder Störung.

13. Verbindung nach einem der Ansprüche 1 bis 10 zum Gebrauch bei der Behandlung einer durch einen α7-Nikotin-Acetylcholinrezeptor (nAChR) vermittelten Krankheit oder Störung.

14. Verbindung zum Gebrauch nach Anspruch 12 oder Anspruch 13, wobei die Behandlung für ein Vorbeugen und/oder Unterdrücken von Husten, einem Fortschreiten von Husten, für ein Lindern von Hustensymptomen und für ein Linden des Wiederauftretens von Husten dient.

15. Verfahren für eine Herstellung einer Verbindung der allgemeinen Formel (I), wie dargestellt in Schema (I): 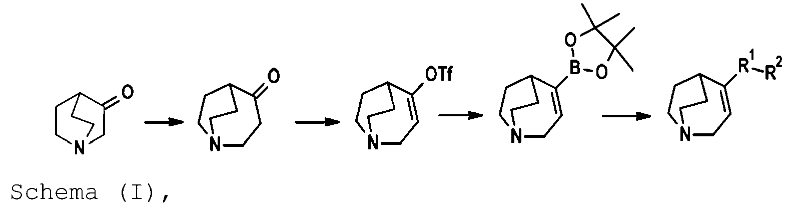 wobei R¹ und R² wie in beliebigen der Ansprüche 1 bis 14 definiert sind.

## Revendications

1. Composé de formule générale (I) : 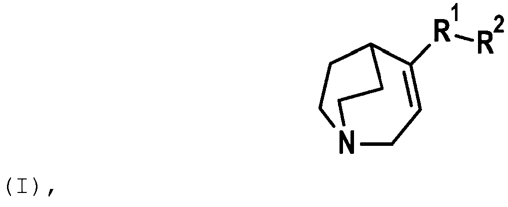
ou sel pharmaceutiquement acceptable de celui-ci,
dans lequel
R¹ est un aryle éventuellement substitué ou un hétéroaryle éventuellement substitué ; et
R² est un halogène, un triflate, un aryle éventuellement substitué, un hétéroaryle éventuellement substitué ou est absent.

2. Composé selon la revendication 1, dans lequel le ou chaque aryle ou hétéroaryle est un cycle de 5 à 10 chaînons, de préférence un cycle de 5 chaînons ou un cycle de 6 chaînons.

3. Composé selon la revendication 1 ou la revendication 2, dans lequel le ou chaque groupe aryle ou hétéroaryle est monocyclique.

4. Composé selon la revendication 3, dans lequel le ou chaque groupe aryle ou hétéroaryle est choisi indépendamment dans le groupe constitué de : phényle, furanyle, pyrrolyle, thiényle, pyridinyle, pyrimidinyle, pyridazinyle, pyrazinyle, oxazolyle, oxadiazolyle, isoxazolyle, pyrazolyle, imidazolyle, thiazolyle, isothiazolyle et thiadiazolyle.

5. Composé selon la revendication 1 ou la revendication 2, dans lequel le ou chaque groupe aryle ou hétéroaryle est polycyclique.

6. Composé selon la revendication 5, dans lequel le ou chaque groupe aryle ou hétéroaryle est choisi indépendamment dans le groupe constitué de : naphtalényle, anthracényle, indolizinyle, indolyle, isoindolyle, benzofuranyle, benzothiophényle, benzodioxolanyle, indazolyle, pyrrolopyridinyle, benzimidazolyle, benzothiazolyle, benzoisothiazolyle, purinyle, thiénopyrazinyle, quinolinyle, isoquinolinyle, cinnolinyle, phtalazinyle, quinazolinyle, quinoxalinyle, 1,8-naphtyridinyle, ptéridinyle, carbazolyle, acridinyle, naphtiridinyle, phénazinyle, phénothiazinyle, phénoxazinyle et azulényle.

7. Composé selon l'une quelconque des revendications précédentes, dans lequel le ou chaque groupe aryle ou hétéroaryle est substitué, de préférence avec 1, 2 ou 3 substituants, dans lequel éventuellement les substituants sont choisis indépendamment dans le groupe constitué de : alkyle, alcényle, hétérocyclyle, cycloalkyle, aryle, aryle substitué, arylalkyle, arylalkyle substitué, halo (de préférence, F, Cl, Br ou I), --OR', --NR'R'', -CF3, --CN, --NO₂, --SR', --N₃, -C(=O)NR'R'', --NR'C(=O)R'', -C(=O)R', --C(=O)OR', --OC(=O)R', --O(CR'R''), --C(=O)R', --SO₂R' et --SO₂NR'R'', dans lequel R' et R'' sont individuellement hydrogène, alkyle inférieur (de préférence, alkyle à chaîne droite ou ramifiée comportant C₁-C₈, de préférence C₁-C₅, tel que méthyle, éthyle ou isopropyle), cycloalkyle, hétérocyclyle, aryle ou arylalkyle (tel que benzyle), et dans lequel R' et R'' se combinent éventuellement pour former une fonctionnalité cyclique.

8. Composé selon la revendication 7, dans lequel les substituants sont choisis indépendamment dans le groupe constitué de : cyano, halogéno, alkyle, halogénoalkyle, cycloalkyle, alcoxy, halogénoalcoxy et alkylthio.

9. Composé selon l'une quelconque des revendications précédentes, dans lequel R¹ est choisi parmi : 1,3,4-thiadiazolyle, pyridinyle, pyridazinyle, pyrimidinyle et pyrazinyle, et/ou dans lequel R² est choisi parmi indolyle, benzofuranyle, benzothiazolyle et phényle.

10. Composé selon l'une quelconque des revendications précédentes, dans lequel le composé est choisi dans le groupe constitué de :
4-(3-pyridyl)-1-azabicyclo[3.2.2]non-3-ène ;
4-(6-phényl-3-pyridyl)-1-azabicyclo[3.2.2]non-3-ène ; et
4-(6-phénylpyridazin-3-yl)-1-azabicyclo[3.2.2]non-3-ène,
de préférence dans lequel le composé est 4-(6-phényl-3-pyridyl)-1-azabicyclo[3.2.2]non-3-ène.

11. Composition pharmaceutique comprenant un composé selon l'une quelconque des revendications 1 à 10 et un excipient ou véhicule pharmaceutiquement ou thérapeutiquement acceptable.

12. Composé selon l'une quelconque des revendications 1 à 10, destiné à être utilisé dans le traitement d'une maladie ou d'un trouble.

13. Composé selon l'une quelconque des revendications 1 à 10, destiné à être utilisé dans le traitement d'une maladie ou d'un trouble médié par un récepteur nicotinique α7 de l'acétylcholine (nAChR) .

14. Composé destiné à être utilisé selon la revendication 12 ou la revendication 13, dans lequel le traitement est destiné à prévenir et/ou à supprimer la toux, la progression de la toux, à améliorer les symptômes de la toux et à améliorer la récurrence de la toux.

15. Procédé de fabrication d'un composé de formule générale (I) telle que représentée dans le schéma (I) : 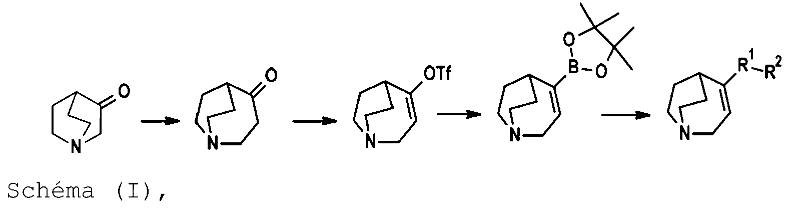 dans lequel R¹ et R² sont tels que définis dans l'une quelconque des revendications 1 à 14.
